# EUROPEAN PATENT APPLICATION

(11) **EP 1 611 875 A1**
(43) Date of publication of application: **04.01.2006**
(21) Application number: 04726629.1
(22) Date of filing: 08.04.2004
(51) Int. Cl.: A61K 6/00

(54) **DENTAL ADHESIVE AND DENTAL FILLER**

(30) Priority: 09.04.2003 JP 2003105437
(71) Applicant: Um Dental Co., Ltd., Tsu-shi, Mie 514-0062 (JP)
(72) Inventor: UEYAMA, Norikazu, Osaka-shi, Osaka 554-0012 (JP); MOHRI, Hiroshi, Noda-shi, Chiba 278-0015 (JP)
(74) Representative: Vuillermoz, Bruno
(86) International application number: PCT/JP2004/005080
(87) International publication number: WO 2004/089314

(57) **Abstract**

A dental adhesive composition includes an organic polymer that contains a unit (A) containing a (substituted) carboxyl group, which is represented by formula (I), and a unit (B) containing a (substituted) carbamoyl group, which is represented by a formula (II). In the organic polymer, the sum of the two units (A) and (B) accounts for at least 20 mol% of all the units that constitute the organic polymer, and the ratio of unit (A) / unit (B) is within a range from 0.6/1.0 to 1.0/0.6. When the quantity of the unit having a smaller quantity than the other unit in the units (A) and (B) within the polymer is deemed 100 mol%, then in at least 70 mol% of the unit having a smaller quantity, the carbon bonded to the above (substituted) carboxyl group and the carbon bonded to the above (substituted) carbamoyl group are either directly adjacent, or bonded together via a methylene group or ethylene group.

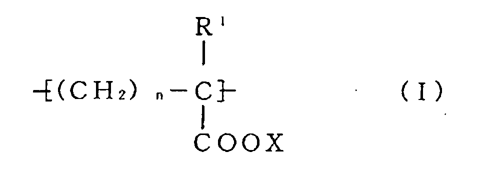

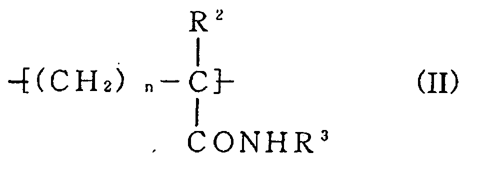

## Description

### TECHNICAL FIELD

The present invention relates to a dental adhesive and a dental filler.

Priority is claimed on Japanese Patent Application No. 2003-105437, filed April 9, 2003, the content of which is incorporated herein by reference.

### BACKGROUND ART

In conventional dental treatment, a variety of techniques and treatment methods have been used for ensuring favorable bonding between the tooth substrate and the restorative dental material.

Considerable progress has also been made in the materials used, from restoration using luting materials such as silicate cements, through to restoration using acrylic resins or composite resins. However, secondary caries, the dislodging of prostheses and the like, caused by unsatisfactory adhesion between the restorative dental material and the tooth substrate, has been identified as an ongoing problem.

In the 1970s and 1980s, the concept of adhering the restorative dental material to the tooth substrate was introduced. As a result, a variety of adhesion techniques that enable adhesion to enamel and dentin have been developed.

Of these, the technique that has been adopted within the dental field as the most reliable technique is a method that uses a monomer with an adhesive function. Namely, a technique such as that described below.
1) The tooth substrate is impregnated with a low molecular weight functional monomer which has a functional group that bonds with the Ca in the tooth substrate.
2) A monomer that is capable of copolymerization with the low molecular weight functional monomer is used in combination with the low molecular weight functional monomer to effect a copolymerization and form a tough resin layer.
3) This resin layer functions as a protective layer at the interface between the living tissue and the artificial material, thereby blocking external stimuli as well as performing an effective role in integrating the tooth substrate and the artificial material through adhesive bonding.

Together with improvements in etching and primer techniques, this technique has contributed considerably to modem dental care, and is one of the core dental treatments now used.

Until now, the most effective examples as this low molecular weight functional monomer have been 1) carboxylic acid-based monomers which have a carboxyl group at the terminal end or the anhydrides thereof, typified by 4-methacryloyloxyethyl trimellitate anhydride (4-META). Other examples thereof include 4-methacryloyloxyethyl trimellitic acid (4-MET), 4-acryloyloxyethyl trimellitate anhydride (4-AETA), and ω,ω-dicarboxydodecyl methacrylate (MAC-10) (for example, see Japanese Examined Patent Application, Second Publication No. 58-17513).

One other effective type of the low molecular weight functional monomer is 2) phosphate ester-based monomers which have a phosphate group at a terminal end and/or a non-terminal end position within the molecule, typified by 2-methacryloyloxyethyl phosphoric acid (2-MEP), and other examples of this type of monomer include 2-methacryloyloxyethyl hydrogen phenyl phosphate (Phenyl-P) and 10-methacryloyloxydecyl dihydrogen phosphate (MDP) (for example, see Japanese Examined Patent Application, Second Publication No. 55-30768).

However, although the dental adhesives disclosed in the above Patent Applications are used in actual teeth treatments for bonding tooth substrates to restorative dental materials such as metal or ceramic materials, the adhesion is still not entirely satisfactory. Namely, inadequate adhesive strength or deterioration in the adhesive layer over time can cause trouble resulting from secondary caries and/or dislodging of prostheses, and the need for retreatment is a fairly frequent occurrence.

Furthermore, the filling of pits and the like within tooth substrates using a polymerizable monomer that includes a filler, and subsequent polymerization of the monomer, is a technique that is currently employed, and from the viewpoint of preventing dislodging of the filler, a material that exhibits both excellent properties as a dental filler, and excellent adhesion to the tooth substrate is keenly sought. However, the adhesion of conventional dental fillers to the tooth substrate is still not satisfactory.

The causes of the aforementioned secondary caries, and particularly dentinal caries, are thought to be as follows. Namely, the diffusion of the low molecular weight functional monomer to the dentin is unsatisfactory, meaning a gap forms between the healthy dentin and the hybrid layer (resin layer) which is produced following polymerization. It is thought that moisture originating from the dentin that enters this gap causes a gradual deterioration in the adhesive layer over time.

One cause of this type of phenomenon is the fact that the adhesion between the adhesive monomer used and the tooth substrate, that is, the calcium within the tooth substrate, is a low bonding strength adhesion which is based mainly on ion reactions.

Furthermore, when these adhesive monomers are used for dentin, the monomer tends to penetrate into the interior of the sponge-like dentin. As a result, a hybrid layer that coats only the surface portion of the dentin cannot be formed. It is believed that this causes the formation of the protective layer to be unsatisfactory.

### DISCLOSURE OF INVENTION

The present invention improves on these drawbacks associated with conventional dental adhesives, with an object of providing a dental adhesive composition and a dental filler that exhibit excellent adhesive strength to tooth substrates.

A dental adhesive composition of the present invention contains an organic polymer described below. Namely, an organic polymer comprises a unit (A) containing a (substituted) carboxyl group, which are represented by formula (I) shown below, and a unit (B) containing a (substituted) carbamoyl group, which are represented by formula (II) shown below, wherein a sum of the two units (A) and (B) accounts for at least 20 mol% of all units that constitute the organic polymer, a ratio of the unit (A) / unit (B) is within a range from 0.6/1.0 to 1.0/0.6, and when the quantity of the unit (A) or (B) having a smaller quantity than the other unit within the polymer is deemed 100 mol%, then in at least 70 mol% of the unit (A) or (B), the carbon bonded to the (substituted) carboxyl group in the unit (A), and the carbon bonded to the (substituted) carbamoyl group in the unit (B) are either directly adjacent, or bonded together via a methylene group or ethylene group.

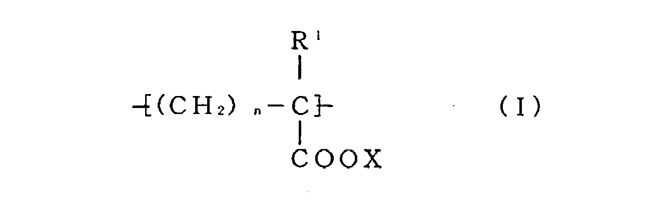

In the formula (I), n represents either 0 or 1, X represents a hydrogen atom, -NH₄, or 1/mM (wherein, M is a metal atom selected from the group consisting of the alkali metals, alkali earth metals, transition metals, Zn and Cd, and m represents the valency of the metal), and R¹ represents a hydrogen atom or a methyl group.

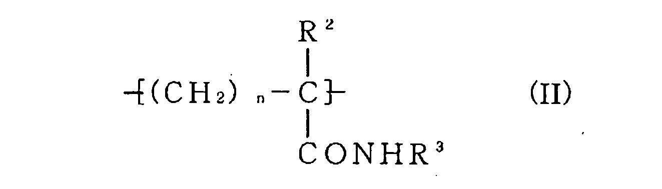

In the formula (II), n represents either 0 or 1, R² represents a hydrogen atom or a methyl group, and R³ represents a hydrogen atom, an alkyl group, alkenyl group, aralkyl group or phosphonoxyalkyl group of 1 to 18 carbon atoms.

Furthermore, a dental filler of the present invention includes the organic polymer described above, a copolymerizable monomer, a filler, and a curing agent.

In the present invention, a (substituted) carboxyl group refers to a carboxyl group, and/or a carboxyl group in which the hydrogen atom has been substituted with -NH₄ or a metal atom selected from the group consisting of the alkali metals, alkali earth metals, transition metals, Zn, and Cd. A (substituted) carbamoyl group refers to a carbamoyl group, and/or a carbamoyl group in which one of the hydrogen atoms has been substituted with an alkyl group, alkenyl group, or aralkyl group of 1 to 18 carbon atoms.

### BEST MODE FOR CARRYING OUT THE INVENTION

As follows is a description of preferred examples of the present invention. However, the present invention is not limited by any of the following examples, and may include, for example, suitable combinations of structural elements from the various examples.

As a result of investigations aimed at resolving the aforementioned problems associated with conventional dental adhesives, the inventors of the present invention discovered that an organic polymer in which carboxyl groups and carbamoyl groups exist in close proximity can bond strongly to materials containing metals such as calcium. By applying this organic polymer to the field of dental adhesives, the inventors were able to complete the present invention. It is surmised that this strong bond is formed as a result of the oxygen atom of the -C=O group of the carboxyl group and the hydrogen atom of the carbamoyl group forming an intramolecular hydrogen bond, thereby strengthening the acid strength of the carboxyl group, and enabling the carboxyl group to form a complex via coordination with the calcium within the tooth substrate. The above hydrogen bond is believed to be formed by a COO⁻ anion derived from the carboxyl group. Furthermore, the term "(substituted)" used above indicates the presence of a substituent (which is deemed to also include the case of a hydrogen atom as a substituent).

The organic polymer used in a dental adhesive composition or dental filler of the present invention is a polymer in which the sum of the two units (A) and (B) is at least 20 mol%, and preferably at least 40 mol%, and even more preferably 60 mol% or more, of all the units that constitute the polymer. Of the various possible polymers, a polymer formed solely from the units (A) and (B), namely, a polymer in which the above sum is 100 mol%, is the most preferred. When the sum of the units (A) and (B) is less than 100%, then there are no particular restrictions on the other units that may be incorporated within the organic polymer. Any appropriate units may be incorporated. Examples of these other units include alkylene units, cycloalkylene units, and units with amide linkages, each of which may contain an alkyl group, an aromatic group, and/or an alkyloxycarbonyl group as a side chain.

The units (A) and (B) must exist within the organic polymer in a ratio (A) : (B) within a range from 0.6:1.0 to 1.0:0.6. Provided the sum of the units (A) and (B) accounts for at least 20 mol% of the polymer, and the ratio (A) : (B) falls within the required range listed above, a structure (C) such as that shown in a formula (III) below, wherein the carbon bonded to the (substituted) carboxyl group of the unit (A), and the carbon bonded to the (substituted) carbamoyl group of the unit (B) are either directly adjacent, or bonded together via a methylene group or ethylene group, can be formed efficiently. When this type of structure (C) is formed, the bonding between the organic polymer and inorganic compounds strengthens, meaning the adhesion between a dental adhesive or dental filler containing the polymer and a tooth substrate also strengthens. In order to ensure the formation of large quantities of this structure (C), the quantities of the two units exiting within the polymer are preferably substantially equal.

The ratio (A) : (B) is preferably from 0.7:1.0 to 1.0:0.7, and even more preferably from 0.8:1.0 to 1.0:0.8. Polymers in which the respective quantities are equal are also very desirable. 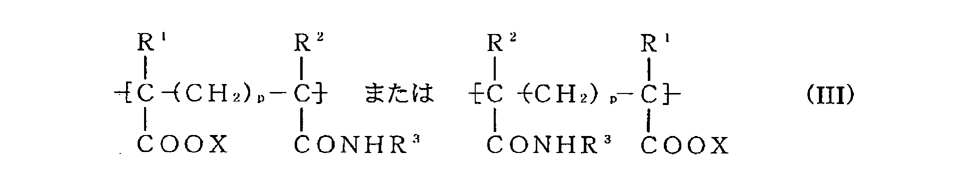 (In the formula (III), p represents 0, 1, or 2, X represents a hydrogen atom, -NH₄, or 1/mM (wherein, M is a metal atom selected from the group consisting of the alkali metals, alkali earth metals, transition metals, Zn and Cd, and m represents the valency of the metal), R¹ represents a hydrogen atom or a methyl group, R² represents a hydrogen atom or a methyl group, and R³ represents an alkyl group, alkenyl group, aralkyl group or phosphonoxyalkyl group of 1 to 18 carbon atoms.)

In addition, when the quantity of one of units (A) and (B) within the polymer is deemed 100 mol% and said one unit exists in smaller amounts than the other within the polymer, then in at least 70 mol%, and preferably in at least 80%, and even more preferably in at least 90%, and most preferably in 100%, of the one unit, the carbon bonded to the carboxyl group in the unit (A) and the carbon bonded to the carbamoyl group in the unit (B) must form a structure (C), wherein the two carbon atoms are either directly adjacent, or bonded together via a methylene group or ethylene group.

For example, in the case in which the units (A) account for 40 mol%, and the units (B) account for 30 mol%, of the 100 mol% of units that constitute the polymer, the quantity of the unit (B), which is the lesser of the two units, is used as the standard. The above requirement means that at least 70 mol% of the units (B) (namely, at least 21 mol% of all the structural units) must exist adjacent to a unit (A), forming a structure (C).

Provided the structure (C) is formed in the above quantities, the adhesion between the dental adhesive composition or dental filler containing the polymer and the tooth substrate is strengthened.

Furthermore, the organic polymer used in the present invention preferably does not exhibit an irregular configuration in which the structures (C) are concentrated within one portion of the polymer and totally absent within other portions, but rather, preferably employs a production method such as that described below to ensure that the structures (C) are distributed comparatively evenly throughout the polymer molecular chain. It is thought that if the structures (C) are distributed evenly in this manner, then the dental adhesive composition or dental filler containing the polymer should bond even more strongly to the tooth substrate. If the organic polymer is produced using the method described below, then the structures (C) can be distributed comparatively evenly through the polymer molecule.

In the structure (C), the oxygen atom of the -C=O group of the (substituted) carboxyl group and the hydrogen atom of the (substituted) carbamoyl group form an intramolecular hydrogen bond. It is thought that the existence of this hydrogen bond strengthens the acid strength of the carboxyl group, enabling powerful bonding to the calcium within the tooth substrate.

For example, examples of benzoic acids which do not have a carbamoyl group in close proximity to the carboxyl group include the following compounds. Namely, the pKa value for 2,2-dimethylpropanoylaminobenzoic acid is 5.4, and the pKa value for 2,4,6-trimethylbenzoic acid, which exhibits steric hindrance, is 4.8. In contrast, 2-(2,2-dimethylpropanoyl)-6-methylbenzoic acid, which has a carbamoyl group in close proximity to the carboxyl group, has a stronger acid value, with a pKa value of 3.9, and 2,6-di(2,2-dimethylpropanoyl)methylbenzoic acid, which has carbamoyl groups on both sides of the carboxyl group, has an even stronger acid value, with a pKa value of 3.1. It is thought that these observations reflect the fact that when a carbamoyl group exists in the vicinity of the carboxyl group, an intramolecular hydrogen bond is formed, and the formation of this hydrogen bond causes an increase in the acid strength of the carboxyl group.

In terms of promoting this hydrogen bonding and increasing the acid strength, R³ is preferably an alkyl group, alkenyl group, or aralkyl group.

In terms of the ease of formation of the aforementioned intramolecular hydrogen bond, the value of p in the structure (C) is preferably either p = 0 (wherein the carbon bonded to the (substituted) carboxyl group of the unit (A), and the carbon bonded to the (substituted) carbamoyl group of the unit (B) are directly adjacent to one another) or p = 1 (wherein the carbon bonded to the (substituted) carboxyl group of the unit (A), and the carbon bonded to the (substituted) carbamoyl group of the unit (B) are bonded together via a methylene group). A single organic polymer molecule may include simultaneously a structure for which p = 0, a structure for which p = 1, and a structure for which p = 2.

The degree of polymerization of the organic polymer is preferably at least 5, and even more preferably 10 or greater, and even more preferably 50 or more, and most preferably 100 or more. A higher degree of polymerization means that when an adhesive layer is formed through adhesion with the tooth substrate, that adhesive layer forms as a tough resin layer that functions effectively as a protective layer for blocking external stimuli at the interface between the living tissue and the artificial material provided thereon. Furthermore, from the viewpoints of ease of preparation of the dental adhesive or dental filler, and production execution ability, the degree of polymerization of the organic polymer is preferably no more than 10,000. Here, the term "degree of polymerization" refers to the number average degree of polymerization.

For example, an organic polymer used in a dental adhesive of the present invention can be produced by the method described below.

First, using a carboxyl group-containing monomer such as (meth)acrylic acid, a carboxyl group-containing polymer in which (meth)acrylic acid units account for at least 20 mol% of all the units that constitute the polymer is produced by normal methods.

Subsequently, the thus obtained carboxyl group-containing polymer is treated with a dehydration agent, thereby converting adjacent sets of 2 carboxyl groups into acid anhydride structures, and generating a polymer with introduced acid anhydride groups.

These acid anhydride groups are then subjected to a ring-opening addition under conventional conditions, using an amidation agent such as ammonia or an alkylamine, thereby forming monoamide groups. This reaction can generate an organic polymer that contains structures (C), wherein the carbon bonded to a (substituted) carboxyl group and the carbon bonded to a (substituted) carbamoyl group are either directly adjacent, or bonded together via a methylene group or ethylene group.

When the structure (C) is formed after polymerization of a carboxyl group-containing monomer such as (meth)acrylic acid, it is envisaged that, during polymerization, the aforementioned formula (III) may include cases of head-head (tail-tail) bonding as well as cases of head-tail bonding. Those cases in which p is either 0 or 2 represent cases of head-head (tail-tail) bonding, whereas the case in which p = 1 represents head-tail bonding.

In an alternative method, a polymer is formed by conventional methods using maleic anhydride, and by subsequently conducting a ring-opening addition of the acid anhydride groups within the thus formed polymer under conventional conditions, using an amidation agent such as ammonia or an amine, an organic polymer that can be used in a dental adhesive composition or dental filler of the present invention can be produced.

Because this organic polymer is synthesized entirely from a monomer containing an acid anhydride group, such as maleic anhydride, the value of p in the resulting structure (C) is 0; that is, the carbon atom bonded to the (substituted) carboxyl group, and the carbon atom bonded to the (substituted) carbamoyl group must be bonded together directly within the main polymer chain.

Accordingly, when synthesizing the organic polymer, provided a monomer containing an acid anhydride group is used, structures (C) in which p is 0 can be produced with certainty.

In the polymerization of a monomer containing an acid anhydride group, another monomer capable of copolymerization with maleic anhydride can also be used.

In polymers using maleic anhydride, the maleic anhydride unit becomes the unit (III). When provided maleic anhydride accounts for at least 70% of the polymer, the polymer may be a random copolymer. However, if the quantity of maleic anhydride is within a range from 40% to less than 70%, then in order to ensure that the structures (C) are distributed evenly, an alternating copolymer in which the maleic anhydride portions are distributed evenly is preferred.

An alternating copolymer can be obtained by a radical polymerization of maleic anhydride and an electron-donating monomer.

Electron-donating monomers are monomers for which the characteristic e-value is negative. Examples thereof include allyl monomers such as allyl alcohol, vinyl ether monomer such as methyl vinyl ether and ethyl vinyl ether, cyclic ether monomers such as p-dioxene, vinyl ester monomers such as vinyl acetate, α-olefins such as propylene, and styrenes.

The adhesive component of a dental adhesive of the present invention may contain only the organic polymer described above, but may also include other polymerizable monomers and/or curing agents in addition to the organic polymer. In those cases where the adhesive component contains only an aforementioned organic polymer, a solution formed by dissolving the polymer in an organic solvent can be used for the adhesion process. In those cases where a polymerizable monomer is included, a solution formed, for example, by dissolving the organic polymer and a curing agent and the like in the polymerizable monomer can be used for the adhesion process.

A dental adhesive of the present invention includes an organic polymer described above, and this organic polymer bonds strongly to the tooth substrate and also can exhibit favorable affinity for polymerizable monomers. Accordingly, due to the dental adhesive, the penetration of these polymerizable monomers into the interior of the tooth substrate can be inhibited, and a resin layer is formed at the surface of the tooth substrate and the layer functions as a protective layer for blocking external stimuli.

This protective layer undergoes minimal hydrolysis over time, wherein hydrolysis can cause a deterioration in adhesion, meaning the dislodging of composite resins or prostheses that have been bonded using the adhesive can be prevented over long periods of time.

A dental filler of the present invention includes an organic polymer described above, a polymerizable monomer, a filler, and a curing agent. If this type of dental filler is used to fill a pit within a tooth, and is subsequently cured (by polymerization of the monomer), then the organic polymer bonds strongly to the tooth, and a polymer formed by polymerization of the monomer is entwined strongly with the organic polymer, with the filler embedded therein. Accordingly, the filler is prevented from dislodging from the pit, and exhibits excellent properties as a dental filler.

As a result, the filler can be bonded strongly to the tooth substrate without the use of an adhesive between the tooth substrate and the filler. A dental filler of the present invention may, of course, also be used following application of a dental adhesive.

The types of polymerizable monomers that can be used in dental adhesives and dental fillers according to the present invention include any of the polymerizable monomers that are typically used in dental fillers. These monomers can be used either alone, or in combinations of two or more different monomers. However, normally a polyfunctional monomer, or a combination of a polyfunctional monomer and a monofunctional monomer, is used. Specific examples of these polymerizable monomers include polyfunctional monomers, including aromatic methacrylic acid esters such as bisphenol A dimethacrylate, bisphenol A diglycidyl methacrylate, 2,2-bis(4-methacryloyloxyethoxyphenyl) propane, and 2,2-bis(4-methacryloyloxypropoxyphenyl) propane; and polyfunctional methacrylic acid esters such as ethylene glycol dimethacrylate, triethylene glycol dimethacrylate, neopentyl glycol dimethacrylate, and trimethylolpropane trimethacrylate; as well as monofunctional monomers such as methyl methacrylate and 2-hydroxyethyl methacrylate.

The filler used in the present invention may also use any filler typically used in dental fillers. These fillers can be used either alone, or in combinations of two or more different fillers. Specific examples of these fillers include silica powder, glass beads, aluminum oxide powder, and quartz powder. The filler is preferably surface treated with a silane coupling agent. Surface treatment with a silane coupling agent can be conducted by a conventional method, using a conventional silane coupling agent.

There are no particular restrictions on the curing agent which can be used in a dental adhesive or dental filler of the present invention, provided it is capable of causing curing at room temperature within a period of several minutes. The curing agent can be used either alone, or in combinations of two or more different curing agents. Examples of suitable curing agents include camphorquinone, combinations such as amine-peroxide combinations, p-toluenesulfinic acid-peroxide combinations, and trialkyl boron-peroxide combinations, and photosensitizers.

Conventional materials can be used as these curing agents.

If required, one or more other polymers such as polymethylmethacrylate, adhesion imparting agents, polymerization accelerators, polymerization regulators, polymerization inhibitors and the like may also be added to a dental filler of the present invention.

There are no particular restrictions on the respective blend ratios within a dental filler of the present invention, and these can be adjusted appropriately in accordance with the intended use. However, typical blend ratios are from 40 to 90% by mass for the filler, from 2 to 20% by mass for the organic polymer, from 5 to 55% by mass for the polymerizable monomer, from 0 to 10% by mass of polymethylmethacrylate, and from 0.5 to 5 parts by mass of the curing agent per 100 parts by mass of the polymerizable monomer.

In those cases where a dental adhesive composition of the present invention is used as an adhesive for bonding a filler to a tooth substrate, a solution containing at least 2% by mass of an aforementioned organic polymer dissolved in a solvent and/or a polymerizable monomer can be used as the adhesive. The solvent may be any appropriate solvent, and suitable examples include ethanol, ethyl ether, chloroform, methylene dichloride, acetone, and methyl ethyl ketone. These solvents can be used either alone, or in combinations. In those cases where a polymerizable monomer is used, the composition preferably also contains a curing agent. The polymerizable monomer and curing agent can use the same materials disclosed in the description of the composition of the filler.

### EXAMPLES

As follows is a more detailed description of the present invention using a series of examples, although the present invention is in no way limited to these examples.

### (Example 1)

2.16 g of polyacrylic acid (Mw: 450,000) was dissolved in 100 ml of methanol, 5.56 g of tri-n-butylamine was added at room temperature under constant stirring, and the mixture was then stirred for a further 12 hours. Subsequently, the methanol was removed at 50°C under reduced pressure conditions, using an evaporator, and the residue was then dissolved in 200 ml of dichloromethane. 5.21 g of phosphorus nitride chloride cyclic trimer was added to this solution at 30°C under constant stirring, and the mixture was then stirred for 12 hours. Subsequently, 200 ml of diethyl ether was added to the solution, thereby precipitating a solid.

The solid was isolated by filtration, washed with a small quantity of dichloromethane, and then dried, yielding 1.6 g of a polyacrylic anhydride. As shown below, the generation of an acid anhydride group was confirmed by IR analysis.
IR (KBr): 1804 cm⁻¹ (C=O), 1760 cm⁻¹ (C=O), 1030 cm⁻¹ (CO-O-CO)

0.64 g of the polyacrylic anhydride was added to 8.78 g of t-butylamine at room temperature, and the resulting mixture was stirred for 12 hours. Following evaporation to a dry solid using an evaporator, the solid was dissolved in 10 g of water. Concentrated hydrochloric acid was then added dropwise to the solution to adjust the pH to 2 to 3. The precipitated solid was collected by filtration, and washed with diethyl ether. The results of subsequent elemental analysis and ¹H-NMR analysis are shown below.
Elemental analysis: (C₁₀H₁₇NO₃)
Calculated values: C 60.28 mol%, N 7.03 mol%, H 8.60 mol%
Measured values: C 60.52 mol%, N 7.12 mol%, H 7.82mol%
¹H-NMR (DMSO-d₆): 12.00 ppm (COOH), 6.9 ppm (CONH), 1.17 ppm (CH₃)

From these results it was determined that an organic polymer A had been obtained in which acrylic acid unit and N-t-butylacrylamide unit were arranged alternately.

Integration of the ¹H-NMR chart revealed a ratio between -COOH and -CONH within the organic polymer A of 1.00:0.88. Furthermore, under these reaction conditions it is believed that 100 mol% of the amide-based units of the polymer A have formed a structure (C).

10 parts by mass of the thus obtained organic polymer A was diluted with 90 parts by mass of 2-hydroxyethyl methacrylate, and camphorquinone was then added in a quantity equivalent to 1% by mass relative to the 2-hydroxyethyl methacrylate, thus forming a solution (solution A).

Furthermore, another solution (solution B) was prepared by adding camphorquinone to a 1:1 mixture of 2-hydroxyethyl methacrylate and methyl methacrylate, in a quantity equivalent to 1 % by mass relative to the combined mass of the 2-hydroxyethyl methacrylate and methyl methacrylate.

The coronal portions of bovine teeth were ground horizontally, and the surfaces were polished with a #600 waterproof abrasive paper to prepare two types of samples: one type in which the enamel was exposed, and one type in which the dentin was exposed. These samples were immersed in water for at least one day, and were then removed from the water and wiped free of moisture immediately prior to testing. The aforementioned solution A was applied to the surface of each of these teeth samples and dried, the solution B was then further applied over the top of the dried surface, and an acrylic rod of diameter 5 mm was then placed on top of each sample and irradiated with visible light to effect curing and adhesion.

Following curing, each sample was subjected to 5000 repetitions of a thermal recycling test in which the temperature was cycled between 5°C and 55°C, with each temperature held for 30 seconds. Subsequently, using a universal tester (product name: Autograph, manufactured by Shimadzu Corporation), the tensile adhesive strength was determined at a crosshead speed of 1 mm/minute.

The results are shown in Table 1.

### (Comparative Example 1)

A composition prepared by adding a small quantity of polymethylmethacrylate to a mixed solution containing 95% by mass of methyl methacrylate, 5% by mass of 4-methacryloyloxyethyl trimellitate anhydride, and oxidized tri-n-butylborane as a polymerization initiator was applied to bovine teeth samples similar to those used in the example 1, and the methyl methacrylate was polymerized at room temperature to bond an acrylic rod of diameter 5 mm to each bovine tooth sample. Each of these acrylic rod-adhered bovine teeth samples was subjected to the same thermal recycling test as that conducted in the example 1, and the tensile adhesive strength was then measured in the same manner as the example 1. The results are shown in Table 1.

### (Example 2)

Acrylic rod-adhered bovine teeth samples prepared in the same manner as the example 1 were immersed in water at 55°C for 2 weeks, and the tensile adhesive strength was then measured in the same manner as the example 1. The results are shown in Table 1.

### (Comparative Example 2)

Acrylic rod-adhered bovine teeth samples prepared in the same manner as the comparative example 1 were immersed in water at 55°C for 2 weeks, and the tensile adhesive strength was then measured in the same manner as the example 1. The results are shown in Table 1.

### (Example 3)

40 parts by mass of 2,2-bis(4-(2-hydroxy-3-methacryloyloxypropoxy)phenyl) propane and 27 parts by mass of triethylene glycol dimethacrylate were used as polymerizable monomers, and 0.2 parts by mass of camphorquinone and 0.2 parts by mass of 4-dimethylaminobenzoic acid were used as polymerization initiators. 10 parts by mass of the organic polymer A prepared in the example 1 was added to the polymerizable monomers together with the polymerization initiators, thus preparing a matrix monomer. Next, this matrix monomer and 100 parts by mass of a fine powder of silane-treated silica as a filler were placed in an agate mortar and ground well in a dark environment to form a uniform paste, thus completing preparation of a dental composite resin.

Bovine teeth samples were prepared in the same manner as the example 1, by exposing the enamel in one type of sample and the dentin in the other type, immersing the samples in water for at least one day, and then removing the samples and wiping away any residual moisture. A silicone rubber mold of internal diameter 5.0 mm and height 2.0 mm was placed on the exposed enamel or dentin of each bovine tooth sample, and the dental composite resin obtained above was then injected into the mold and cured by irradiation with light, thus forming a test specimen in each case. These test specimens were allowed to stand for 24 hours in a thermostatic chamber at a temperature of 37°C and a relative humidity of 100%. Subsequently, a tensile-testing acrylic rod was mounted to the upper portion of each test specimen, and a universal tester (product name: Autograph, manufactured by Shimadzu Corporation) was used to determine the tensile adhesive strength at a crosshead speed of 1 mm/minute. The results are shown in Table 1.

### (Comparative Example 3)

With the exception of replacing the organic polymer A from the example 3 with a similar quantity of the polymerizable monomer (a 40:27 (mass ratio) mixture of 2,2-bis(4-(2-hydroxy-3-methacryloyloxypropoxy)phenyl) propane and triethylene glycol dimethacrylate), sample preparation and determination of the tensile adhesive strength were conducted in the same manner as the example 2. The results are shown in Table 1.

**Table 1**

| | Tensile adhesive strength (Mpa) (n = 10) | |
|---|---|---|
| | Enamel | Dentin |
| Example 1 | 14.8 ± 2.0 | 11.2 ± 2.6 |
| Comparative example 1 | 12.1 ± 4.5 | 8.1 ± 3.4 |
| Example 2 | 14.2 ± 2.9 | 10.5 ± 3.9 |
| Comparative example 2 | 6.2 ± 3.9 | 3.9 ± 2.5 |
| Example 3 | 12.4 ± 3.2 | 10.3 ± 3.3 |
| Comparative example 3 | 0 | 0 |

As is evident from Table 1, although the adhesive of the comparative example 1, which used 4-META, exhibits reasonable adhesion, with values of 12.1 MPa for adhesion to enamel and 8.1 MPa for adhesion to dentin, these values are still not entirely satisfactory. In contrast, the adhesives of the present invention exhibit excellent adhesion to both enamel and dentin.

Furthermore, as is clear from a comparison of the comparative examples 1 and 2, conventional dental adhesives suffer a dramatic reduction in adhesive strength on immersion in water at 55°C, whereas a similar comparison of the examples 1 and 2 reveals that the reduction in adhesive strength for a dental adhesive of the present invention is minimal. It is surmised that this observation reflects the fact that whereas conventional dental adhesives undergo hydrolysis of the adhesive on immersion in water, dental adhesives of the present invention do not undergo hydrolysis.

Furthermore, whereas a conventional dental filler (composite resin) exhibits no adhesion to the tooth substrate when applied directly to either the enamel or the dentin without any adhesive treatment, a dental filler of the present invention exhibits favorable adhesion, even on direct filling of the composite resin without any adhesive pretreatment.

### INDUSTRIAL APPLICABILITY

The present invention provides a dental adhesive composition and a dental filler that exhibit strong bonding to tooth substrates. According to the present invention, a dental adhesive composition and dental filler can be provided that exhibit a higher adhesive strength than has conventionally been attainable. Furthermore, a protective layer formed by a dental adhesive of the present invention suffers minimal hydrolysis over time, which can cause a deterioration in adhesion, and thus provides excellent dental treatment effects.

## Claims

1. A dental adhesive composition comprising an organic polymer, wherein
the organic polymer comprises a unit (A) containing a (substituted) carboxyl group, which is represented by formula (I) below, and a unit (B) containing a (substituted) carbamoyl group, which is represented by formula (II) below;
a sum of the units (A) and (B) accounts for at least 20 mol% of all units that constitute the organic polymer;
a ratio of the unit (A) / unit (B) in the polymer is within a range from 0.6/1.0 to 1.0/0.6; and
when the quantity of the unit (A) or (B) having a smaller quantity than the other unit within the polymer is deemed 100 mol%, then in at least 70 mol% of the unit (A) or (B), a carbon bonded to the (substituted) carboxyl group in the unit (A), and a carbon bonded to the (substituted) carbamoyl group in the unit (B) are either directly adjacent, or bonded together via a methylene group or ethylene group 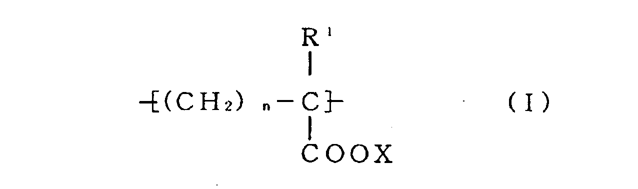 (in formula (I), n represents either 0 or 1, X represents a hydrogen atom, -NH₄, or 1/mM (wherein, M is a metal atom selected from the group consisting of alkali metals, alkali earth metals, transition metals, Zn and Cd, and m represents a valency of the metal), and R¹ represents a hydrogen atom or a methyl group) 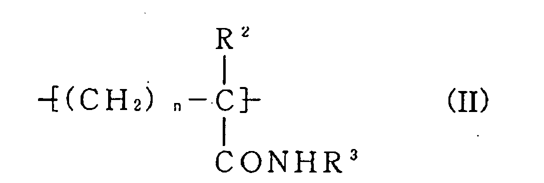 (in formula (II), n represents either 0 or 1, R² represents a hydrogen atom or a methyl group, and R³ represents a hydrogen atom, an alkyl group, alkenyl group, aralkyl group or phosphonoxyalkyl group of 1 to 18 carbon atoms).

2. A dental adhesive composition according to claim 1, wherein a sum of the units (A) and (B) accounts for at least 40 mol% of all units that constitute the organic polymer.

3. A dental adhesive composition according to claim 1, wherein the ratio of the unit (A) / unit (B) is within a range from 0.7/1.0 to 1.0/0.7.

4. A dental adhesive composition according to claim 1, wherein when the quantity of the unit (A) or (B) having a smaller quantity than the other unit within the polymer is deemed 100 mol%, then in at least 80 mol% of the unit (A) or (B), a carbon bonded to the (substituted) carboxyl group in the unit (A), and a carbon bonded to the (substituted) carbamoyl group in the unit (B) are either directly adjacent, or bonded together via a methylene group or ethylene group.

5. A dental adhesive composition according to claim 1, wherein R³ is at least one group selected from the group consisting of alkyl groups, alkenyl groups, and aralkyl groups.

6. A dental filler comprising an organic polymer, a copolymerizable monomer, a filler and a curing agent, wherein
the organic polymer comprises a unit (A) containing a (substituted) carboxyl group, which is represented by formula (I) below, and a unit (B) containing a (substituted) carbamoyl group, which is represented by formula (II) below;
a sum of the units (A) and (B) accounts for at least 20 mol% of all units that form the organic polymer;
a ratio of the unit (A) / unit (B) in the organic polymer is within a range from 0.6/1.0 to 1.0/0.6; and
when the quantity of the unit (A) or (B) having a smaller quantity than the other unit within the polymer is deemed 100 mol%, then in at least 70 mol% of the unit (A) or (B), a carbon bonded to the (substituted) carboxyl group in the unit (A), and a carbon bonded to the (substituted) carbamoyl group in the unit (B) are either directly adjacent, or bonded together via a methylene group or ethylene group 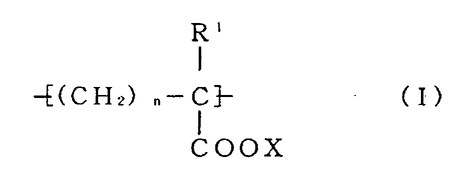
(in the formula (I), n represents either 0 or 1, X represents a hydrogen atom, -NH₄, or 1/mM (wherein, M is a metal atom selected from the group consisting of alkali metals, alkali earth metals, transition metals, Zn and Cd, and m represents a valency of the metal), and R¹ represents a hydrogen atom or a methyl group) 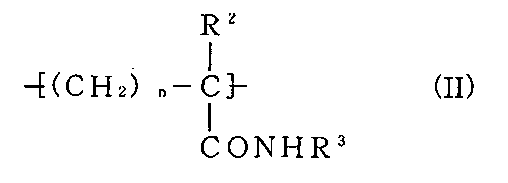 (in the formula (II), n represents either 0 or 1, R² represents a hydrogen atom or a methyl group, and R³ represents a hydrogen atom, an alkyl group, alkenyl group, aralkyl group or phosphonoxyalkyl group of 1 to 18 carbon atoms).

7. A dental filler according to claim 6, wherein a sum of the units (A) and (B) accounts for at least 40 mol% of all units that constitute the organic polymer.

8. A dental filler according to claim 6, wherein the ratio of unit (A) / unit (B) is within a range from 0.7/1.0 to 1.0/0.7.

9. A dental filler according to claim 6, wherein when the quantity of the unit (A) or (B) having a smaller quantity than the other unit within the polymer is deemed 100 mol%, then in at least 80 mol% of the unit (A) or (B), a carbon bonded to the (substituted) carboxyl group in the unit (A), and a carbon bonded to the (substituted) carbamoyl group in the unit (B) are either directly adjacent, or bonded together via a methylene group or ethylene group.

10. A dental filler according to claim 6, wherein R³ is at least one group selected from the group consisting of alkyl groups, alkenyl groups, and aralkyl groups.
